# EUROPEAN PATENT APPLICATION

(11) **EP 3 730 616 A1**
(43) Date of publication of application: **28.10.2020**
(21) Application number: 18892746.1
(22) Date of filing: 18.12.2018
(51) Int. Cl.: C12N 15/113, C12N 9/22, C12N 15/85

(54) **SPLIT SINGLE-BASE GENE EDITING SYSTEMS AND APPLICATION THEREOF**

(30) Priority: 18.12.2017 CN 201711368309
(71) Applicant: East China Normal University, Shanghai 200241 (CN); Bioray Laboratories Inc, Shanghai 200241 (CN)
(72) Inventor: LI, Dali, Shanghai 200241 (CN); ZHANG, Xiaohui, Shanghai 200241 (CN); WANG, Liren, Shanghai 200241 (CN); ZHU, Biyun, Shanghai 200241 (CN); CHEN, Liang, Shanghai 200241 (CN); LIU, Mingyao, Shanghai 200241 (CN)
(74) Representative: Mewburn Ellis LLP
(86) International application number: PCT/CN2018/121779
(87) International publication number: WO 2019/120193

(57) **Abstract**

Provided are two split single-base gene editing systems. Intein-mediated split BE3, saKKH-BE3 and ABE7.10 are separately developed by using the existing protein structure information of spCas9 and saCas9 and splitting methods thereof, and have targeted gene mutation efficiency equivalent to that of unsplit BE3, saKKH-BE3 and ABE7.10 working system, thereby making possible package into an AAV for delivery.

## Description

### Technical field

The present invention relates to the field of biotechnology, in particular, to a split- single base gene editing system and the application thereof.

### Background

Since 2013, a new generation of gene editing technology represented by CRISPR / Cas9 has entered various labs in the field of biology and is changing the traditional methods of gene manipulation.

At present, the single-base gene editing technology has been reported to be used for efficient gene mutation or repair in genome, generation of disease animal model and gene therapy. Among the found single-base gene editing tools, BE3, SaKKH-BE3, and ABE7.10, are the most widely used. However, BE3, SaKKH-BE3, and ABE7.10 has a length of 5.1kb, 4.3kb, and 5.3kb, respectively. With the addition of the promoter and polyA elements, it has exceeded the 4.7kb packaging limit of the adeno-associated virus (AAV), which prevents them from being packaged into AAV for delivery and hinders the widespread use in gene editing, gene therapy and clinical practice.

Therefore, there is an urgent need in the art to develop a split single-base gene editing system that can maintain a considerable targeted gene mutation efficiency.

### Summary of the invention

The object of the present invention is to provide a split single-base gene editing system that can maintain a considerable targeted gene mutation efficiency.

The object of the present invention is also to provide a split single-base gene editing system (BE3, SaKKH-BE3, ABE7.10), which is split into N-terminal and C-terminal, respectively, the length of which is less than the adeno-associated virus (AAV) packaging limit of 4.7Kb and can be packaged and delivered with AAV, further expanding the single-base gene editing technology, especially in the field of gene therapy.

In a first aspect of the present invention, it provides a nucleic acid construct combination comprising a first nucleic acid construct and a second nucleic acid construct, wherein the first nucleic acid construct has a structure of Formula I from 5'-3':

P1-X1-X2-X3-Z-X4 (I);

wherein PI is a first promoter sequence;
X1 is a coding sequence of cytosine deaminase or a coding sequence of adenosine deaminase;
X2 is an optional linker sequence;
X3 is a coding sequence of the N-terminal fragment of Cas9 nuclease or a coding sequence of the N-terminal fragment of SaKKH nuclease (Cas9n-N or SaKKH-N);
Z is a coding sequence of the N-terminal fragment of a first fusion peptide;
X4 is a polyA sequence;
the second nucleic acid construct has a structure of Formula II from 5'-3 ':

P2-Z-Y1-Y2-Y3 (II);

wherein P2 is a second promoter sequence;
Z is a coding sequence of the C-terminal fragment of a first fusion peptide;
Y1 is a coding sequence of the C-terminal fragment of Cas9 nuclease or a coding sequence of the C-terminal fragment of SaKKH nuclease (Cas9n-C or SaKKH-C);
Y2 is a coding sequence of UGI or none;
Y3 is a polyA sequence;
and each "-" is independently a bond or a nucleotide linker sequence.

In another preferred embodiment, if X1 is a coding sequence of adenosine deaminase, Y2 is none.

In another preferred embodiment, the first promoter and the second promoter are each independently selected from the group consisting of: a CAG promoter, CMV promoter, and a combination thereof.

In another preferred embodiment, the first promoter sequence comprises a CMV promoter.

In another preferred embodiment, the second promoter sequence comprises a CMV promoter.

In another preferred embodiment, the linker sequence includes XTEN, GGS, (GGS) 3, (GGS) 7.

In another preferred embodiment, the cytosine deaminase includes Apobecl.

In another preferred embodiment, the adenosine deaminase is derived from a bacteria, human, rat, and/or mouse.

In another preferred embodiment, the Cas9 nuclease is selected from the group consisting of Cas9, Cas9n, and a combination thereof.

In another preferred embodiment, the N-terminal fragment of Cas9n is amino acids 2-573 of Cas9n nuclease (Accession Number (Gene ID): 2828055).

In another preferred embodiment, the C-terminal fragment of Cas9n is amino acids 574-1368 of Cas9n nuclease (Accession Number (Gene ID): 2828055).

In another preferred embodiment, the SaKKH nuclease is from *Staphylococcus.*

In another preferred embodiment, the N-terminal fragment of SaKKH is amino acids 2-739 of SaKKH nuclease (Accession Number (Gene ID): 2828033).

In another preferred embodiment, the C-terminal fragment of SaKKH is amino acids 740-1053 of SaKKH nuclease.

In another preferred embodiment, the origin of the X3 element is selected from the group consisting of *Streptococcus pyogenes, Staphylococcus aureus,* and a combination thereof.

In another preferred embodiment, in the X3 element, the mutation site is at position D10A of Cas9n-N (SEQ ID NO.: 1).

In another preferred embodiment, in the X3 element, the mutation site is at position D10A of SaKKH-N (SEQ ID NO.: 2).

In another preferred embodiment, in the Y1 element, there is no mutation site in Cas9n-C (SEQ ID NO.: 4).

In another preferred embodiment, in the Y1 element, the mutation site is at position E782K/N968K/R1015H of SaKKH-C (SEQ ID NO.: 3).

In another preferred embodiment, the polyA sequence is each independently selected from the group consisting of BGH polyA, SV40 polyA, and a combination thereof.

In another preferred embodiment, the length of the first nucleic acid construct is ≤4.7 kb, preferably, ≤4.5 kb, and more preferably, 3.0-4.5 kb.

In another preferred embodiment, the length of the second nucleic acid construct is ≤4.7 kb, preferably, ≤4.5 kb, and more preferably, 3.0-4.5 kb.

In another preferred embodiment, the N-terminal fragment of the first fusion peptide and the C-terminal fragment of the first fusion peptide together constitute an active first fusion peptide.

In another preferred embodiment, the first fusion peptide is selected from the group consisting of intein, FRB/FKBP, DmC/FKBP, ABI/PYL, and a combination thereof.

In another preferred embodiment, the N-terminal fragment of the first fusion peptide is amino acids 2-103 of the fusion peptide (such as an intein).

In another preferred embodiment, the C-terminal fragment of the first fusion peptide is amino acids 104-137 of the fusion peptide (e.g., intein).

In a second aspect of the present invention, it provides a vector combination, comprising a first vector and a second vector, the first vector contains a first nucleic acid construct, the second vector contains the second nucleic acid construct, the first nucleic acid construct and the second nucleic acid construct are as defined in the first aspect of the present invention.

In another preferred embodiment, the first vector and the second vector are viral vectors.

In another preferred embodiment, the first vector and the second vector are AAV viral vectors.

In another preferred embodiment, in the first vector, the first construct is located in an expression cassette with inverted terminal repeats at both ends of the first vector.

In another preferred embodiment, in the second vector, the second construct is located in an expression cassette with inverted terminal repeats at both ends of the second vector.

In another preferred embodiment, the vector combination includes a first viral vector and a second viral vector, preferably, the first viral vector and the second viral vector are both AAV viral vectors.

In another preferred embodiment, the genetically engineered cell is a prokaryotic cell or a eukaryotic cell.

In another preferred embodiment, the prokaryotic cell includes: E. coli.

In another preferred embodiment, the eukaryotic cell is selected from the group consisting of a yeast cell, plant cell, mammalian cell (such as a HEK293T cell), human cell, and a combination thereof.

In another preferred embodiment, the genetically engineered cell is prepared by the first viral vector and the second viral vector through viruses.

In a fourth aspect of the present invention, it provides a method for gene editing in a cell, comprising the steps of:
(i) providing a cell and a first vector and a second vector, wherein the first vector contains a first nucleic acid construct, the second vector contains a second nucleic acid construct, the first nucleic acid construct and the second nucleic acid construct are as defined in the first aspect of the present invention, and both the first vector and the second vector are viral vectors;
(ii) infecting the cell with the viral vector, thereby performing gene editing in the cell.

In another preferred embodiment, in step (ii), the method further comprises simultaneously infecting the cell with a third vector encoding gRNA.

In another preferred embodiment, the first vector and/or the second vector.

In another preferred embodiment, the method is non-diagnostic and non-therapeutic.

In another preferred embodiment, the gene editing comprises: site-specific cleavage, site-specific insertion, and site-specific recombination.

In another preferred embodiment, the cell is from the following species: a human, non-human mammal, poultry, plant.

In another preferred embodiment, the non-human mammal includes a rodent (such as a mouse, rat, rabbit), cow, pig, sheep, horse, dog, cat, and non-human primate (such as a monkey).

In another preferred embodiment, the cell includes: a somatic cell, stem cell, germ cell, non-dividing cell, and a combination thereof.

In another preferred embodiment, the cell includes: a kidney cell, epithelial cell, endothelial cell, and a combination thereof.

In a fifth aspect of the present invention, it provides a kit, comprising:
(a1) a first container, and a first vector located in the first container; and
(b1) a second container, and a second vector located in the second container.

In another preferred embodiment, the first container and the second container are different containers.

In another preferred embodiment, the kit further includes instructions that describe a method of infecting a cell with the first vector and the second vector to perform gene editing in the cell.

In another preferred embodiment, the kit further contains (c1) a third container, and a third container containing a third vector encoding gRNA.

In another preferred embodiment, the first vector and/or the second vector.

It should be understood that, within the scope of the present invention, the technical features specifically described above and below (such as the Examples) can be combined with each other, thereby constituting a new or preferred technical solution which needs not be described one by one.

### Description of Drawings

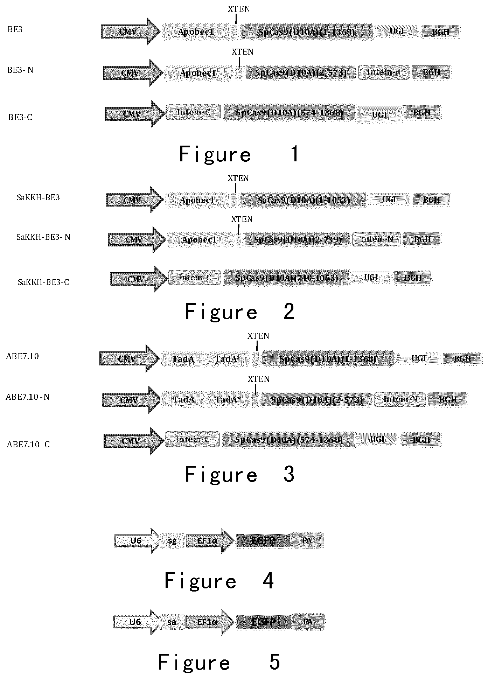

Figure 1 shows a schematic diagram of BE3 and split-BE3(BE3-N, BE3-C). Wherein CMV: promoter; U6: human derived U6 promoter; Apobec1 : cytosine deaminase ; XTEN: linker; SpCas9n: D10A mutant nicked SpCas9 ; SpCas9(D10A)(2-573) represents amino acids 2-573 of SpCas9 with a nick of D10A mutation; SpCas9(D10A)(574-1368) represents amino acids 574-1368 of SpCas9 with a nick of D10A mutation; UGI: uracil glycosidase inhibitor; BGH:PolyA sequence.
Figure 2 shows a schematic diagram of SaKKH-BE3 and the split SaKKH-BE3. Wherein CMV: promoter; U6: human derived U6 promoter; Apobec1 : cytosine deaminase; XTEN: linker; SaCas9n(KKH): in addition to the D10A mutation, it also contains the E782K/N968K/R1015H amino acid mutation; SaCas9(D10A)(2-739) represents amino acids 2-739 of SpCas9 with a nick of D10A mutation;SaCas9(D10A)(740-1053 represents amino acids 740-1053 of SpCas9 with a nick of D10A mutation; UGI: uracil glycosidase inhibitor; BGH: PolyA sequence.
Figure 3 shows a schematic diagram of ABE7.10 and split-ABE7.10 (ABE7.10-N, ABE7.10-C). Wherein CMV: promoter; U6: human derived U6 promoter; Apobec1: adenosine deaminase; XTEN: linker; SpCas9n: D10A mutated nicked SpCas9; SpCas9 (D10A) (2-573) represents amino acids 2-573 of SpCas9 with a nick of D10A mutation; SpCas9 (D10A) (574-1368) represents amino acids 574-1368 of SpCas9 with a nick of D10A mutation; BGH: PolyA sequence.
Figure 4 a schematic diagram of U6-spsgRNA-EF1α-GFP. Wherein U6: human derived U6 promoter; sg: represents the insertable SpCas9 target; EF1α: promoter: EGFP : Enhanced Green Fluorecence Protein; PA: PolyA sequence.
Figure 5 shows a schematic diagram of U6-sasgRNA-EF1α-GFP. Wherein U6: human derived U6 promoter; sg: represents the insertable SaCas9 target;; EF1α: promoter: EGFP : Enhanced Green Fluorecence Protein; PA: PolyA sequence.

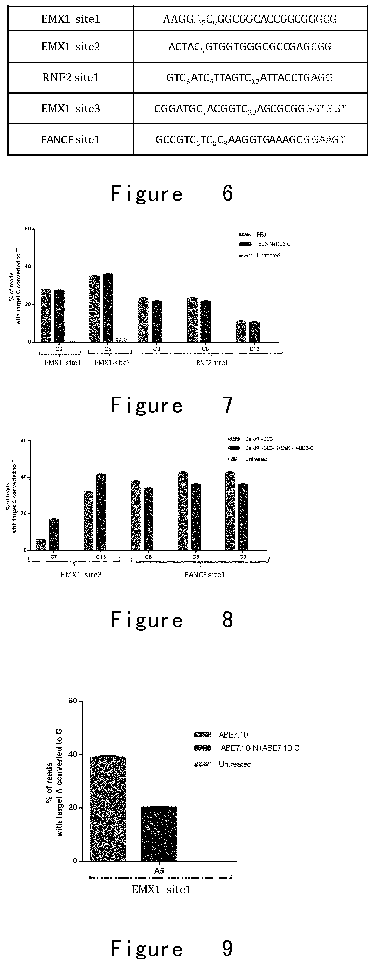

Figure 6 shows a list of tested targets.
Figure 7 shows the comparison of the mutation efficiency of BE3 and split BE3 for endogenous gene targets EMX1 site1, EMX1 site2, RNF2 site1. Among them, the ordinate: representing the percentage of single base C to T mutation; the abscissa: representing C at different positions of 3 endogenous targets of EMX1 site1, EMX1 site2, RNF2 site1.
Figure 8 shows the comparison of the mutation efficiency of SaKKH and the split SaKKH for the endogenous genes EMX1 site3 and FANCF site1. Among them, the ordinate: represents the percentage of single base C to T mutation; the abscissa: represents C at different positions of 2 endogenous targets of EMX1 site3, FANCF site 1.
Figure 9 shows the comparison of the mutation efficiency of ABE7.10 and split ABE7.10 for the endogenous gene target EMX1 site1. Among them, the ordinate: represents the percentage of single base A to G mutation; the abscissa: represents C at different positions of an endogenous target of EMX1 site1..

### DETAILED DESCRIPTION OF INVENTION

After extensive and intensive research, the inventors has first time found that intein-mediated resolution of BE3, SaKKH-BE3 and ABE7.10 were developed respectively using the existing protein structure information of spCas9 and saCas9 and their splits methods, and the split BE3, SaKKH, ABE7.10 has a higher target gene mutation efficiency, which is comparable to the target gene mutation efficiency of the unsplit BE3, SaKKH, ABE7.10 working system, providing the possibility of packaging into AAV for delivery, and promoting its wide application in gene editing, gene therapy and clinical. On this basis, the inventor has completed the present invention.

### BE3 working system

BE3, that is Base editor 3, which is formed by fusion of cytosine deaminase and spCas9 (spCas9n) with a D10A mutation derived from *Streptococcus pyogenes.* It uses NGG as PAM and recognizes and specifically binds DNA and a single base mutation from C to T is achieved at positions 16-19 upstream of NGG (**Figure 1**).

### SaKKH-BE3 working system

SaKKH is formed by fusion of cytosine deaminase and SaCas9 with a D10A mutation and saCas9 with E782K/N968K/R1015H mutation from *Staphylococcus aureus* with NNNRRT (R=A or G) as PAM and recognize and specifically bind DNA and realize C to T single base mutation at positions 11-16 upstream of PAM (Figure 2)..

### ABE7.10 working system

ABE7.10, which is formed by fusion of adenosine deaminase with spCas9 (spCas9n) with a D10A mutation derived from *Streptococcus pyogenes.* It uses NGG as PAM and recognizes and specifically binds DNA and realizes a single-base mutation from A to G at positions 16-19 upstream of NGG (Figure 3).

### Self-cleaving protein

Self-cleaving protein 2A is a class of 18-22 amino acid peptides. When it connects two or more proteins, its translated protein product can be cleaved between glycine and proline(Asp-Val/Ile-Glu-X-Asn-Pro-Gly-↓-Pro) at highly conserved C-terminal of 2A. Therefore, the protein products at both ends of 2A can be functioned independently. The self-cleaving protein used in this invention is T2A derived from tetrahymenops β-larvae virus (Thosea asignavirus). There are also F2A from foot-and-mouth disease virus, E2A from horse rhinitis virus, and P2A from swine Jieshen 1 virus.

In a preferred embodiment, the self-cleaving protein is a 2A sequence. The 2A sequence is from the virus and is a short peptide of 18-22 amino acids. It expresses multiple proteins in an open reading frame through self-splicing, and the self-splicing efficiency is almost 100%. Commonly used are T2A, P2A , F2A, E2A.

### Fusion peptide

In the present invention, the fusion peptide is not particularly limited, and a preferred fusion peptide is selected from the group consisting of intein, FRB / FKBP, DmC / FKBP, ABI / PYL, and a combination thereof.

In a preferred embodiment, the fusion peptide is an intein. Intein is a naturally occurring intermediate sequence, which can catalyze protein splicing reaction to make inteins and flanking peptides into tandems with natural peptide bonds. Inteins control enzyme activity at specific times based on splicing reactions and coupling of peptides from different sources.

### Construct combination of the present invention

The invention provides a nucleic acid construct combination, including a first construct and a second construct. It uses the existing protein structure information of spCas9 or saCas9 and their split methods, intein-mediated split BE3, SaKKH-BE3 and the split BE3, SaKKH-BE3 and ABE7.10 were developed respectively. Among them, the split BE3 and SaKKH-BE3 nucleic acid construct can achieve the C mutation to T in the endogenous gene target, which is equivalent to the targeted gene mutation efficiency of the unsplit BE3 and SaKKH-BE3 working systems. Splitting the ABE7.10 nucleic acid construct can achieve the A mutation to G in the endogenous gene target, compared with the unsplit ABE7.10, the efficiency is slightly lower. The split-type BE3, SaKKH-BE3, and ABE7.10 provide the possibility of being packaged into AAV for delivery, and promote its wide application in base editing, gene therapy and clinical. The construct combination of the invention is as described in the first aspect of the invention.

The various elements used in the construct combination of the invention are known in the field, so those technician in the field can use conventional methods, such as PCR method, fully artificial chemical synthesis method, and enzyme digestion method to obtain the corresponding elements. These elements were ligased together by well-known DNA ligation techniques and then the construct combination of the invention is formed.

Specifically, the construction, verification process and application of the construct combination in the invention are as follows:
1. According to the human EMX1, RNF2, FANCF gene, BE3, SaKKH-BE3 and ABE7.10 target sequence were designed respectively (Figure-6), and its sgRNA oligo was designed according to its target (Table 1). According to structure information of Spcas9 and saCas9, using intein as fusion linker peptide, the split BE3 was designed, Splitting occurs between amino acids 573 and 574 of Spcas9, that is BE3-N, BE3-C (Figure-1), at the same time, the split SaKKH-BE3 was designed and splitting occurs between amino acids 739 and 740 of Sacas9 (Figure-2), that is SaKKH-BE3-N and SaKKH-BE3-C (Figure-2). At the same time, the split ABE7.10 was designed, splitting occurs between amino acids 573 and 574 of Spcas9, namely ABE7.10-N, ABE7.10-C (Figure-3). Simultaneously the sgRNA vector U6-spsgRNA-EF1α-GFP with spCas9 sacffold (Figure 4) was designed and expressed and the sgRNA vector U6-sasgRNA-EF1α-GFP with saCas9 sacffold (Figure 5) was expressed. At the same time, according to the working principle of CRISPR / Cas9, the plasmids in Figure-4 and Figure-5 were digested with BbsI, and then ligased to the corresponding annealed sgRNA oligo (Table-1).
2. The above plasmids in sequence was constructed.
3. Comparing the fixed-point editing efficiency of C to T for BE3 and BE3-N/BE3-C, SaKKH-BE3 and SaKKH-BE3-N/SaKKH-BE3-C on human endogenous gene targets (Figure 6); comparing the fixed-point editing efficiency of A to G for ABE7.10 and ABE7.10-N/ABE7.10-C on human endogenous gene targets (Figure-6).

That is, the following combination was co-transfected with 293T,
BE3: U6-sgRNA-EF1α-GFP= 750ng:250ng,
BE3-N: BE3-C: U6-sgRNA-EF1α-GFP=375 ng:375 ng:250ng
SaKKH-BE3: U6-sgRNA-EF1α-GFP= 750ng:250ng,
SaKKH-BE3-N: SaKKH-BE3-C: U6-sgRNA-EF1α-GFP=375 ng:375ng:250ng
ABE7.10: U6-sgRNA-EF1α-GFP= 750ng:250ng,
ABE7.10-N: ABE7.10-C: U6-sgRNA-EF1α-GFP=375 ng:375 ng:250ng
72h after transfection, GFP positive cells were sorted by flow cytometry. According to the instructions of the HITOM kit (Nuohe Zhiyuan item number: PT026), after performing PCR using the first round of PCR identification primers (Table-2), and then the second round of PCR library construction is completed, high-throughput sequencing was performed, comparing the editing efficiency of endogenous targets for BE3 and split BE3, SaKKH-BE3 and split SaKKH-BE3, ABE7.10 and split ABE7.10 (Figure-7, Figure-8, Figure-9) .

**The results show that BE3 and split BE3, SaKKH-BE3 and split SaKKH-BE3, ABE7.10 and split ABE7.10 have a considerable editing efficiency at the endogenous target.**

**Table 1. oligo design**

| **name** | **sequence** | **SEQ ID NO.:** |
|---|---|---|
| EMX1 site1-up | CACCGAAGGACGGCGGCACCGGCGG | 5 |
| EMX1 site1-dn | AAACCCGCCGGTGCCGCCGTCCTT | 6 |
| EMX1 site2-up | CACCGACTACGTGGTGGGCGCCGAG | 7 |
| EMX1 site2-dn | AAACCTCGGCGCCCACCACGTAGT | 8 |
| RNF2 site1-up | CACCGGTCATCTTAGTCATTACCTG | 9 |
| RNF2 site1-dn | AAACCAGGTAATGACTAAGATGAC | 10 |
| EMX1 site3-up | CACCGCGGATGCACGGTCAGCGCGG | 11 |
| EMX1 site3-dn | AAACCCGCGCTGACCGTGCATCCG | 12 |
| FANCF site1-up | CACCGGCCGTCTCCAAGGTGAAAGC | 13 |
| FANCF site1-dn | AAACGCTTTCACCTTGGAGACGGC | 14 |

**Table 2. target identification primers**

| **name** | **sequence** | **SEQ ID NO.:** |
|---|---|---|
| EMX1 sitel-HITOM-F | GGAGTGAGTACGGTGTGCTCCCGCGGCTGCGACCA | 15 |
| EMX1 sitel-HITOM-R | GAGTTGGATGCTGGATGGGGGTGAGGGTAGTTGAGCGCC | 16 |
| EMX1 site2-HITOM-F | GGAGTGAGTACGGTGTGCCCCTTCGCACGCAAGCCCAAG | 17 |
| EMX1 site2-HITOM-R | GAGTTGGATGCTGGATGGCGGGGGTGATTACCTGCGTCTCG | 18 |
| RNF2 site1-HITOM-F | GGAGTGAGTACGGTGTGCTATTTCCAGCAATGTCTCAGGCT | 19 |
| RNF2 site1-HITOM-R | GAGTTGGATGCTGGATGGGTTTTCATGTTCTAAAAATGTATC CCA | 20 |
| EMX1 site3-HITOM-F | GGAGTGAGTACGGTGTGCCTTCGTGAGTGGCTTCCCTGCC | 21 |
| EMX1 site3-HITOM-R | GAGTTGGATGCTGGATGGGAAGAAGGAGTGCGGGGGCTG | 22 |
| FANCF site1-HITOM-F | GGAGTGAGTACGGTGTGCTCGACCAATAGCATTGCAGAGAG GC | 23 |
| FANCF site1-HITOM-R | GAGTTGGATGCTGGATGGGCTGACGTAGGTAGTGCTTGAGACC | 24 |

**The main advantages of the present invention include:**
(1) In the present invention, for the first time, using the existing protein structure information of spCas9 and saCas9 and the split methods, intein-mediated resolution of BE3, SaKKH-BE3, and ABE7.10 were developed, respectively.Split-BE3, SaKKH-BE3 and ABE7.10 have a significant targeted gene mutation efficiency, which is comparable to the targeted gene mutation efficiency of the unsplit BE3, KKH working system, which provides the possibility of packaging into AAV for delivery and promoting its gene widely used in base editing, gene therapy and clinic.
(2) The split single-base gene editing system of the present invention splits BE3, SaKKH-BE3, ABE7.10 into N-terminal and C-terminal, its length is less than packaging limit (4.7Kb) of the adeno-associated virus (AAV), which can be packaged and delivered with AAV, further expanding the scope of application of the base editor.

The present invention will be further described below in conjunction with specific case. These cases are not only used to illustrate the present invention, but also expande the scope of the present invention. The experimental methods unless specifically stated generally follow conventional conditions such as Sambrook et al. Molecular cloning: the conditions described in the laboratory manual (New York: Cold Spring Harbor Laboratory Press, 1989), or according to the conditions the manufacturer recommended. Unless otherwise stated, percentages and parts are calculated by weight. The experimental materials and reagents involved in the present invention can be obtained from commercially available channels without special instructions.

### Example 1 Comparison of the mutation efficiency of targeting endogenous gene using unsplit and split BE3, SaKKH-BE3, ABE7.10

### 1. Design and construction of split-type split BE3, SaKKH-BE3, ABE7.10 plasmid vector and sgRNA

### 1.1 Design of split BE3, SaKKH-BE3, ABE7.10 plasmid

The BE3 and ABE7.10 splits were all fused with SpCas9 (D10A). According to the structure information of SpCas9, splitting occurs between amino acids 573 and 574 of SpCas9; and SaKKH-BE3 is formed by a fusion of SaCas9, According to the structure information of SaCas9, splitting occurs between amino acids 739 and 740 of SpCas9.

### 1.2 Design principle of sgRNA:

According to the working principle of CRISPR/Cas9 and BE3, KKH, the human EMX1, RNF2, FANCF target sequences were designed (Figure 6), the design principle of target sequence sgRNA oligo is as follows:
In CRISPR/Cas9, spCas9 recognizes PAM (NGG) and SaCas9 recognizes PAM (NNNRRT); and at the same time, 20 bases of complementary paired sgRNA are required for targeted binding. The sgRNA uses U6 as the promoter and requires G as the transcription start site. At the same time, U6-SpsgRNA-EF1α-GFP and U6-SasgRNA-EF1α-GFP are ligased into the target site by BbsI enzyme digestion sites. CACCG was needed to be added at 5' end of sgRNA oligo-up and AAAC was needed to be added at 5' end of sgRNA oligo-up, the specific sequence is designed as follows.

### 1.3 Construction of the plasmid in 1.2

Synthesis of sgRNA oligo.
1.3.1 Dissolving oligo in pure water to a final concentration of 100 µM.
1.3.2 Annealing. 10µL of each of the two complementary oligos were mixed, and put in a boiling water bath for 5min, then cooled naturally to room temperature, about 2 hours.
1.3.3 ligased. U6-SpsgRNA-EF1α-GFP (BbsI) and U6-SasgRNA-EF1α-GFP (BbsI) were digested with BbsI and the vectors and annealed oligo were ligated and reacted according to the following reaction system.

Take Biyuntian's Rapid DNA ligation kit (D7006) as an example.

| | |
|---|---|
| annealed oligo | 1 µL |
| precut vector | 1 µL (10-50ng) |
| T4 DNA Ligase | 1 µL |
| 10x T4 DNA Ligase buffer | 1 µL |
| H2O | add to 10µL |

After ligased at room temperature for 60 min, 5 µL mixture was transformed into 50 µL of competent bacteria, coated with kanamycin-resistant plates, and incubated overnight at 37 ° C.
1.3.4 Two clones was picked from the overnight culture plate, inoculated in a 4-5 mL medium, shaked at 37 ° C incubated overnight at 220r/min.
1.3.5 After shaking culture overnight, the plasmids were extracted and verified by hU6 sequencing, sequencing the correct plasmid..

### 2. Comparison of targeted endogenous gene mutation efficiency of unsplit and split BE3, SaKKH-BE3 and ABE7.10.

### 2.1 Plasmid transfection

### Day 1 Seeding a 24-well plate with 293T cells

2.1.1 The HEK293T cells were digested and seeded a 24-well plate at 2.0 × 10⁵ cell / well.

Note: After the cells were recovered, they generally need to be passaged twice before being used for transfection experiments.

### Day 2 Transfection

2.1.2 The state of cells in each well was observed.
   Note: The cell density before transfection should be 80% -95%, and the condition is normal.
2.1.3 To ensure the accuracy of the data and the repeatability of the experiment, the plasmid was diluted with sterile water. Diluting the plasmid concentration of each group to be consistent, or ensure that the volume of plasmid samples between the groups was the same.
   The group settings were as follows:
   Blank: blank control, including only cultured cells and culture medium;
   The treatment groups were:
      That is the combination of the following was used to transfect 293T,
      BE3: U6-sgRNA-EF1α-GFP= 750ng:250ng,
      BE3-N: BE3-C: U6-sgRNA-EF1α-GFP=375 ng:375 ng:250ng
      SaKKH-BE3: U6-sgRNA-EF1α-GFP= 750ng:250ng,
      SaKKH-BE3-N:SaKKH-BE3-C:U6-sgRNA-EF1α-GFP=375ng:375ng:250ng
      ABE7.10: U6-sgRNA-EF1α-GFP= 750ng:250ng,
      ABE7.10-N: ABE7.10-C: U6-sgRNA-EF1α-GFP=375 ng:375 ng:250ng
2.1.4 Adding DMEM (no serum, no antibiotics) to the 1.5 mL EP tube.
2.1.5 Adding the DNA plasmid to the EP tube in step (4) and mixing it well.
2.1.6 Adding PEI to the EP tube in the previous step, mixing it well, and letting stand at room temperature for 20 minutes.
2.1.7 Adding the transfection mix to the 24-well plate. Gently tapping the 24-well plate to mix well.
2.1.8 After culturing at 37° C, 5% CO₂ for 72h, GFP-positive cells were sorted by flow cytometry.

### Day 5

2.1.9 After 72h, GFP positive cells were sorted by flow cytometry.
2.1.10 Extracting the genomic DNA of the sorted GFP-positive cells with Tiangen Cell Genome Extraction Kit (DP304), and performing PCR using the first round of PCR identification primers (Table-2) according to the instructions of the HITOM kit (Nuohe Zhiyuan Item No.: PT026). After the second round of PCR library construction is completed, high-throughput sequencing were performed. Comparing the editing efficiency of endogenous targets for BE3 and split BE3, SaKKH-BE3 and split SaKKH-BE3, ABE7.10 and split ABE7.10 (Figure-7, Figure-8, Figure-9).

**The results show that BE3 and split BE3, SaKKH-BE3 and split SaKKH-BE3, ABE7.10 and split ABE7.10 have a considerable editing efficiency at each endogenous target.**

### Comparative Example 1

The method of Example 1 was used, except that co-transformation of BE3-N terminal or C terminal with the sgRNA-expressing plasmid, and co-transformation of SaKKH-BE3-N terminal or C terminal with the sgRNA-expressing plasmid and co-transformation of ABE7.10-N terminal or C terminal with the sgRNA-expressing plasmid.

The result shows that none of the endogenous gene mutations are detected.

### Comparative Example 2

The method of Example 1 was used, except that there was no corresponding intein at BE3-N, C-terminal, no corresponding intein at SaKKH-BE3-N, C-terminal, ABE7.10-N or C-terminal, and they were co-transformed with sgRNA plasmid.

The result shows that none of the endogenous gene mutations are detected.

### Comparative Example 3

The method of Example 1 was used, except that BE3-C and SaKKH-BE3-C were not fused with glycosidase inhibitors and co-transformed with sgRNA plasmids.

The result shows that the endogenous gene mutation efficiency is lower, about 10%, and there are also lower unexpected mutations, namely mutation of cytosine to adenine, guanine.

All literatures mentioned in the present application are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. A nucleic acid construct combination comprising a first nucleic acid construct and a second nucleic acid construct, wherein the first nucleic acid construct has a structure of Formula I from 5'-3':
P1-X1-X2-X3-Z-X4 (I);
wherein PI is a first promoter sequence;
X1 is a coding sequence of cytosine deaminase or a coding sequence of adenosine deaminase;
X2 is an optional linker sequence;
X3 is a coding sequence of the N-terminal fragment of Cas9 nuclease or a coding sequence of the N-terminal fragment of SaKKH nuclease (Cas9n-N or SSaKKH-N);
Z is a coding sequence of the N-terminal fragment of a first fusion peptide;
X4 is a polyA sequence;
the second nucleic acid construct has a structure of Formula II from 5'-3 ':
P2-Z-Y1-Y2-Y3 (II);
wherein P2 is a second promoter sequence;
Z is a coding sequence of the C-terminal fragment of a first fusion peptide;
Y1 is a coding sequence of the C-terminal fragment of Cas9 nuclease or a coding sequence of the C-terminal fragment of SSaKKH nuclease (Cas9n-C or SSaKKH-C);
Y2 is a coding sequence of UGI or none;
Y3 is a polyA sequence;
and each "-" is independently a bond or a nucleotide linker sequence.

2. The nucleic acid construct combination of claim 1, wherein the first promoter and the second promoter are each independently selected from the group consisting of: a CAG promoter, CMV promoter, and a combination thereof.

3. The nucleic acid construct combination of claim 1, wherein the Cas9 nuclease is selected from the group consisting of Cas9, Cas9n, and a combination thereof.

4. The nucleic acid construct combination of claim 1, wherein the origin of the X3 element is selected from the group consisting of *Streptococcus pyogenes, Staphylococcus aureus,* and a combination thereof.

5. The nucleic acid construct combination of claim 1, wherein the length of the first nucleic acid construct is ≤4.7 kb, preferably, ≤4.5 kb, and more preferably, 3.0-4.5 kb.

6. The nucleic acid construct combination of claim 1, wherein the length of the second nucleic acid construct is ≤4.7 kb, preferably, ≤4.5 kb, and more preferably, 3.0-4.5 kb.

7. A vector combination, comprising a first vector and a second vector, the first vector contains a first nucleic acid construct, the second vector contains the second nucleic acid construct, the first nucleic acid construct and the second nucleic acid construct are as defined in claim 1.

8. A genetically engineered cell wherein the cell is transformed by the construct of claim 1; or transformed or transfected by the vector combination of claim 7.

9. A method for gene editing in a cell, comprising the steps of:
(i) providing a cell and a first vector and a second vector, wherein the first vector contains a first nucleic acid construct, the second vector contains a second nucleic acid construct, the first nucleic acid construct and the second nucleic acid construct are as defined in claim 1, and both the first vector and the second vector are viral vectors;
(ii) infecting the cell with the viral vector, thereby performing gene editing in the cell.

10. A kit, comprising:
(a1) a first container, and a first vector located in the first container; and
(b1) a second container, and a second vector located in the second container.


<110>  EAST CHINA NORMAL UNIVERSITY
       BIORAY LABORATORIES INC.

<120>  SPLIT SINGLE-BASE GENE EDITING SYSTEMS AND APPLICATION THEREOF

<130>  P2018-2327

<150>  CN201711368309.6
<151>  2017-12-18

<160>  24

<170>  PatentIn version 3.5

<210>  1
<211>  958
<212>  PRT
<213>  Streptococcus pyogenes

<400>  1

Met Asp Tyr Lys Asp His Asp Gly Asp Tyr Lys Asp His Asp Ile Asp
1               5                   10                  15

Tyr Lys Asp Asp Asp Asp Lys Met Ala Pro Lys Lys Lys Arg Lys Val
            20                  25                  30

Gly Ile His Gly Val Pro Ala Ala Ser Ser Glu Thr Gly Pro Val Ala
        35                  40                  45

Val Asp Pro Thr Leu Arg Arg Arg Ile Glu Pro His Glu Phe Glu Val
    50                  55                  60

Phe Phe Asp Pro Arg Glu Leu Arg Lys Glu Thr Cys Leu Leu Tyr Glu
65                  70                  75                  80

Ile Asn Trp Gly Gly Arg His Ser Ile Trp Arg His Thr Ser Gln Asn
                85                  90                  95

Thr Asn Lys His Val Glu Val Asn Phe Ile Glu Lys Phe Thr Thr Glu
            100                 105                 110

Arg Tyr Phe Cys Pro Asn Thr Arg Cys Ser Ile Thr Trp Phe Leu Ser
        115                 120                 125

Trp Ser Pro Cys Gly Glu Cys Ser Arg Ala Ile Thr Glu Phe Leu Ser
    130                 135                 140

Arg Tyr Pro His Val Thr Leu Phe Ile Tyr Ile Ala Arg Leu Tyr His
145                 150                 155                 160

His Ala Asp Pro Arg Asn Arg Gln Gly Leu Arg Asp Leu Ile Ser Ser
                165                 170                 175

Gly Val Thr Ile Gln Ile Met Thr Glu Gln Glu Ser Gly Tyr Cys Trp
            180                 185                 190

Arg Asn Phe Val Asn Tyr Ser Pro Ser Asn Glu Ala His Trp Pro Arg
        195                 200                 205

Tyr Pro His Leu Trp Val Arg Leu Tyr Val Leu Glu Leu Tyr Cys Ile
    210                 215                 220

Ile Leu Gly Leu Pro Pro Cys Leu Asn Ile Leu Arg Arg Lys Gln Pro
225                 230                 235                 240

Gln Leu Thr Phe Phe Thr Ile Ala Leu Gln Ser Cys His Tyr Gln Arg
                245                 250                 255

Leu Pro Pro His Ile Leu Trp Ala Thr Gly Leu Lys Ser Gly Ser Glu
            260                 265                 270

Thr Pro Gly Thr Ser Glu Ser Ala Thr Pro Glu Ser Asp Lys Lys Tyr
        275                 280                 285

Ser Ile Gly Leu Ala Ile Gly Thr Asn Ser Val Gly Trp Ala Val Ile
    290                 295                 300

Thr Asp Glu Tyr Lys Val Pro Ser Lys Lys Phe Lys Val Leu Gly Asn
305                 310                 315                 320

Thr Asp Arg His Ser Ile Lys Lys Asn Leu Ile Gly Ala Leu Leu Phe
                325                 330                 335

Asp Ser Gly Glu Thr Ala Glu Ala Thr Arg Leu Lys Arg Thr Ala Arg
            340                 345                 350

Arg Arg Tyr Thr Arg Arg Lys Asn Arg Ile Cys Tyr Leu Gln Glu Ile
        355                 360                 365

Phe Ser Asn Glu Met Ala Lys Val Asp Asp Ser Phe Phe His Arg Leu
    370                 375                 380

Glu Glu Ser Phe Leu Val Glu Glu Asp Lys Lys His Glu Arg His Pro
385                 390                 395                 400

Ile Phe Gly Asn Ile Val Asp Glu Val Ala Tyr His Glu Lys Tyr Pro
                405                 410                 415

Thr Ile Tyr His Leu Arg Lys Lys Leu Val Asp Ser Thr Asp Lys Ala
            420                 425                 430

Asp Leu Arg Leu Ile Tyr Leu Ala Leu Ala His Met Ile Lys Phe Arg
        435                 440                 445

Gly His Phe Leu Ile Glu Gly Asp Leu Asn Pro Asp Asn Ser Asp Val
    450                 455                 460

Asp Lys Leu Phe Ile Gln Leu Val Gln Thr Tyr Asn Gln Leu Phe Glu
465                 470                 475                 480

Glu Asn Pro Ile Asn Ala Ser Gly Val Asp Ala Lys Ala Ile Leu Ser
                485                 490                 495

Ala Arg Leu Ser Lys Ser Arg Arg Leu Glu Asn Leu Ile Ala Gln Leu
            500                 505                 510

Pro Gly Glu Lys Lys Asn Gly Leu Phe Gly Asn Leu Ile Ala Leu Ser
        515                 520                 525

Leu Gly Leu Thr Pro Asn Phe Lys Ser Asn Phe Asp Leu Ala Glu Asp
    530                 535                 540

Ala Lys Leu Gln Leu Ser Lys Asp Thr Tyr Asp Asp Asp Leu Asp Asn
545                 550                 555                 560

Leu Leu Ala Gln Ile Gly Asp Gln Tyr Ala Asp Leu Phe Leu Ala Ala
                565                 570                 575

Lys Asn Leu Ser Asp Ala Ile Leu Leu Ser Asp Ile Leu Arg Val Asn
            580                 585                 590

Thr Glu Ile Thr Lys Ala Pro Leu Ser Ala Ser Met Ile Lys Arg Tyr
        595                 600                 605

Asp Glu His His Gln Asp Leu Thr Leu Leu Lys Ala Leu Val Arg Gln
    610                 615                 620

Gln Leu Pro Glu Lys Tyr Lys Glu Ile Phe Phe Asp Gln Ser Lys Asn
625                 630                 635                 640

Gly Tyr Ala Gly Tyr Ile Asp Gly Gly Ala Ser Gln Glu Glu Phe Tyr
                645                 650                 655

Lys Phe Ile Lys Pro Ile Leu Glu Lys Met Asp Gly Thr Glu Glu Leu

            660                 665                 670

Leu Val Lys Leu Asn Arg Glu Asp Leu Leu Arg Lys Gln Arg Thr Phe
        675                 680                 685

Asp Asn Gly Ser Ile Pro His Gln Ile His Leu Gly Glu Leu His Ala
    690                 695                 700

Ile Leu Arg Arg Gln Glu Asp Phe Tyr Pro Phe Leu Lys Asp Asn Arg
705                 710                 715                 720

Glu Lys Ile Glu Lys Ile Leu Thr Phe Arg Ile Pro Tyr Tyr Val Gly
                725                 730                 735

Pro Leu Ala Arg Gly Asn Ser Arg Phe Ala Trp Met Thr Arg Lys Ser
            740                 745                 750

Glu Glu Thr Ile Thr Pro Trp Asn Phe Glu Glu Val Val Asp Lys Gly
        755                 760                 765

Ala Ser Ala Gln Ser Phe Ile Glu Arg Met Thr Asn Phe Asp Lys Asn
    770                 775                 780

Leu Pro Asn Glu Lys Val Leu Pro Lys His Ser Leu Leu Tyr Glu Tyr
785                 790                 795                 800

Phe Thr Val Tyr Asn Glu Leu Thr Lys Val Lys Tyr Val Thr Glu Gly
                805                 810                 815

Met Arg Lys Pro Ala Phe Leu Ser Gly Glu Gln Lys Lys Ala Ile Val
            820                 825                 830

Asp Leu Leu Phe Lys Thr Asn Arg Lys Val Thr Val Lys Gln Leu Lys
        835                 840                 845

Glu Asp Tyr Phe Lys Lys Ile Glu Cys Leu Ser Tyr Glu Thr Glu Ile
    850                 855                 860

Leu Thr Val Glu Tyr Gly Leu Leu Pro Ile Gly Lys Ile Val Glu Lys
865                 870                 875                 880

Arg Ile Glu Cys Thr Val Tyr Ser Val Asp Asn Asn Gly Asn Ile Tyr
                885                 890                 895

Thr Gln Pro Val Ala Gln Trp His Asp Arg Gly Glu Gln Glu Val Phe
            900                 905                 910

Glu Tyr Cys Leu Glu Asp Gly Ser Leu Ile Arg Ala Thr Lys Asp His
        915                 920                 925

Lys Phe Met Thr Val Asp Gly Gln Met Leu Pro Ile Asp Glu Ile Phe
    930                 935                 940

Glu Arg Glu Leu Asp Leu Met Arg Val Asp Asn Leu Pro Asn
945                 950                 955

<210>  2
<211>  1125
<212>  PRT
<213>  Staphylococcus aureus

<400>  2

Met Asp Tyr Lys Asp His Asp Gly Asp Tyr Lys Asp His Asp Ile Asp
1               5                   10                  15

Tyr Lys Asp Asp Asp Asp Lys Met Ala Pro Lys Lys Lys Arg Lys Val
            20                  25                  30

Gly Ile His Gly Val Pro Ala Ala Ser Ser Glu Thr Gly Pro Val Ala
        35                  40                  45

Val Asp Pro Thr Leu Arg Arg Arg Ile Glu Pro His Glu Phe Glu Val
    50                  55                  60

Phe Phe Asp Pro Arg Glu Leu Arg Lys Glu Thr Cys Leu Leu Tyr Glu
65                  70                  75                  80

Ile Asn Trp Gly Gly Arg His Ser Ile Trp Arg His Thr Ser Gln Asn
                85                  90                  95

Thr Asn Lys His Val Glu Val Asn Phe Ile Glu Lys Phe Thr Thr Glu
            100                 105                 110

Arg Tyr Phe Cys Pro Asn Thr Arg Cys Ser Ile Thr Trp Phe Leu Ser
        115                 120                 125

Trp Ser Pro Cys Gly Glu Cys Ser Arg Ala Ile Thr Glu Phe Leu Ser
    130                 135                 140

Arg Tyr Pro His Val Thr Leu Phe Ile Tyr Ile Ala Arg Leu Tyr His
145                 150                 155                 160

His Ala Asp Pro Arg Asn Arg Gln Gly Leu Arg Asp Leu Ile Ser Ser
                165                 170                 175

Gly Val Thr Ile Gln Ile Met Thr Glu Gln Glu Ser Gly Tyr Cys Trp
            180                 185                 190

Arg Asn Phe Val Asn Tyr Ser Pro Ser Asn Glu Ala His Trp Pro Arg
        195                 200                 205

Tyr Pro His Leu Trp Val Arg Leu Tyr Val Leu Glu Leu Tyr Cys Ile
    210                 215                 220

Ile Leu Gly Leu Pro Pro Cys Leu Asn Ile Leu Arg Arg Lys Gln Pro
225                 230                 235                 240

Gln Leu Thr Phe Phe Thr Ile Ala Leu Gln Ser Cys His Tyr Gln Arg
                245                 250                 255

Leu Pro Pro His Ile Leu Trp Ala Thr Gly Leu Lys Ser Gly Ser Glu
            260                 265                 270

Thr Pro Gly Thr Ser Glu Ser Ala Thr Pro Glu Ser Gly Lys Arg Asn
        275                 280                 285

Tyr Ile Leu Gly Leu Ala Ile Gly Ile Thr Ser Val Gly Tyr Gly Ile
    290                 295                 300

Ile Asp Tyr Glu Thr Arg Asp Val Ile Asp Ala Gly Val Arg Leu Phe
305                 310                 315                 320

Lys Glu Ala Asn Val Glu Asn Asn Glu Gly Arg Arg Ser Lys Arg Gly
                325                 330                 335

Ala Arg Arg Leu Lys Arg Arg Arg Arg His Arg Ile Gln Arg Val Lys
            340                 345                 350

Lys Leu Leu Phe Asp Tyr Asn Leu Leu Thr Asp His Ser Glu Leu Ser
        355                 360                 365

Gly Ile Asn Pro Tyr Glu Ala Arg Val Lys Gly Leu Ser Gln Lys Leu
    370                 375                 380

Ser Glu Glu Glu Phe Ser Ala Ala Leu Leu His Leu Ala Lys Arg Arg
385                 390                 395                 400

Gly Val His Asn Val Asn Glu Val Glu Glu Asp Thr Gly Asn Glu Leu
                405                 410                 415

Ser Thr Lys Glu Gln Ile Ser Arg Asn Ser Lys Ala Leu Glu Glu Lys
            420                 425                 430

Tyr Val Ala Glu Leu Gln Leu Glu Arg Leu Lys Lys Asp Gly Glu Val
        435                 440                 445

Arg Gly Ser Ile Asn Arg Phe Lys Thr Ser Asp Tyr Val Lys Glu Ala
    450                 455                 460

Lys Gln Leu Leu Lys Val Gln Lys Ala Tyr His Gln Leu Asp Gln Ser
465                 470                 475                 480

Phe Ile Asp Thr Tyr Ile Asp Leu Leu Glu Thr Arg Arg Thr Tyr Tyr
                485                 490                 495

Glu Gly Pro Gly Glu Gly Ser Pro Phe Gly Trp Lys Asp Ile Lys Glu
            500                 505                 510

Trp Tyr Glu Met Leu Met Gly His Cys Thr Tyr Phe Pro Glu Glu Leu
        515                 520                 525

Arg Ser Val Lys Tyr Ala Tyr Asn Ala Asp Leu Tyr Asn Ala Leu Asn
    530                 535                 540

Asp Leu Asn Asn Leu Val Ile Thr Arg Asp Glu Asn Glu Lys Leu Glu
545                 550                 555                 560

Tyr Tyr Glu Lys Phe Gln Ile Ile Glu Asn Val Phe Lys Gln Lys Lys
                565                 570                 575

Lys Pro Thr Leu Lys Gln Ile Ala Lys Glu Ile Leu Val Asn Glu Glu
            580                 585                 590

Asp Ile Lys Gly Tyr Arg Val Thr Ser Thr Gly Lys Pro Glu Phe Thr
        595                 600                 605

Asn Leu Lys Val Tyr His Asp Ile Lys Asp Ile Thr Ala Arg Lys Glu
    610                 615                 620

Ile Ile Glu Asn Ala Glu Leu Leu Asp Gln Ile Ala Lys Ile Leu Thr
625                 630                 635                 640

Ile Tyr Gln Ser Ser Glu Asp Ile Gln Glu Glu Leu Thr Asn Leu Asn
                645                 650                 655

Ser Glu Leu Thr Gln Glu Glu Ile Glu Gln Ile Ser Asn Leu Lys Gly
            660                 665                 670

Tyr Thr Gly Thr His Asn Leu Ser Leu Lys Ala Ile Asn Leu Ile Leu
        675                 680                 685

Asp Glu Leu Trp His Thr Asn Asp Asn Gln Ile Ala Ile Phe Asn Arg
    690                 695                 700

Leu Lys Leu Val Pro Lys Lys Val Asp Leu Ser Gln Gln Lys Glu Ile
705                 710                 715                 720

Pro Thr Thr Leu Val Asp Asp Phe Ile Leu Ser Pro Val Val Lys Arg
                725                 730                 735

Ser Phe Ile Gln Ser Ile Lys Val Ile Asn Ala Ile Ile Lys Lys Tyr
            740                 745                 750

Gly Leu Pro Asn Asp Ile Ile Ile Glu Leu Ala Arg Glu Lys Asn Ser
        755                 760                 765

Lys Asp Ala Gln Lys Met Ile Asn Glu Met Gln Lys Arg Asn Arg Gln
    770                 775                 780

Thr Asn Glu Arg Ile Glu Glu Ile Ile Arg Thr Thr Gly Lys Glu Asn
785                 790                 795                 800

Ala Lys Tyr Leu Ile Glu Lys Ile Lys Leu His Asp Met Gln Glu Gly
                805                 810                 815

Lys Cys Leu Tyr Ser Leu Glu Ala Ile Pro Leu Glu Asp Leu Leu Asn
            820                 825                 830

Asn Pro Phe Asn Tyr Glu Val Asp His Ile Ile Pro Arg Ser Val Ser
        835                 840                 845

Phe Asp Asn Ser Phe Asn Asn Lys Val Leu Val Lys Gln Glu Glu Asn
    850                 855                 860

Ser Lys Lys Gly Asn Arg Thr Pro Phe Gln Tyr Leu Ser Ser Ser Asp
865                 870                 875                 880

Ser Lys Ile Ser Tyr Glu Thr Phe Lys Lys His Ile Leu Asn Leu Ala
                885                 890                 895

Lys Gly Lys Gly Arg Ile Ser Lys Thr Lys Lys Glu Tyr Leu Leu Glu
            900                 905                 910

Glu Arg Asp Ile Asn Arg Phe Ser Val Gln Lys Asp Phe Ile Asn Arg
        915                 920                 925

Asn Leu Val Asp Thr Arg Tyr Ala Thr Arg Gly Leu Met Asn Leu Leu

930                 935                 940

Arg Ser Tyr Phe Arg Val Asn Asn Leu Asp Val Lys Val Lys Ser Ile
945                 950                 955                 960

Asn Gly Gly Phe Thr Ser Phe Leu Arg Arg Lys Trp Lys Phe Lys Lys
                965                 970                 975

Glu Arg Asn Lys Gly Tyr Lys His His Ala Glu Asp Ala Leu Ile Ile
            980                 985                 990

Ala Asn Ala Asp Phe Ile Phe Lys  Glu Trp Lys Lys Leu  Asp Lys Ala
        995                 1000                 1005

Lys Lys  Val Met Glu Asn Gln  Met Phe Glu Glu Lys  Gln Ala Glu
    1010                 1015                 1020

Cys Leu  Ser Tyr Glu Thr Glu  Ile Leu Thr Val Glu  Tyr Gly Leu
    1025                 1030                 1035

Leu Pro  Ile Gly Lys Ile Val  Glu Lys Arg Ile Glu  Cys Thr Val
    1040                 1045                 1050

Tyr Ser  Val Asp Asn Asn Gly  Asn Ile Tyr Thr Gln  Pro Val Ala
    1055                 1060                 1065

Gln Trp  His Asp Arg Gly Glu  Gln Glu Val Phe Glu  Tyr Cys Leu
    1070                 1075                 1080

Glu Asp  Gly Ser Leu Ile Arg  Ala Thr Lys Asp His  Lys Phe Met
    1085                 1090                 1095

Thr Val  Asp Gly Gln Met Leu  Pro Ile Asp Glu Ile  Phe Glu Arg
    1100                 1105                 1110

Glu Leu  Asp Leu Met Arg Val  Asp Asn Leu Pro Asn
    1115                 1120                 1125

<210>  3
<211>  480
<212>  PRT
<213>  Staphylococcus aureus

<400>  3

Met Ile Lys Ile Ala Thr Arg Lys Tyr Leu Gly Lys Gln Asn Val Tyr
1               5                   10                  15

Asp Ile Gly Val Glu Arg Asp His Asn Phe Ala Leu Lys Asn Gly Phe

20                  25                  30

Ile Ala Ser Ser Met Pro Glu Ile Glu Thr Glu Gln Glu Tyr Lys Glu
        35                  40                  45

Ile Phe Ile Thr Pro His Gln Ile Lys His Ile Lys Asp Phe Lys Asp
    50                  55                  60

Tyr Lys Tyr Ser His Arg Val Asp Lys Lys Pro Asn Arg Lys Leu Ile
65                  70                  75                  80

Asn Asp Thr Leu Tyr Ser Thr Arg Lys Asp Asp Lys Gly Asn Thr Leu
                85                  90                  95

Ile Val Asn Asn Leu Asn Gly Leu Tyr Asp Lys Asp Asn Asp Lys Leu
            100                 105                 110

Lys Lys Leu Ile Asn Lys Ser Pro Glu Lys Leu Leu Met Tyr His His
        115                 120                 125

Asp Pro Gln Thr Tyr Gln Lys Leu Lys Leu Ile Met Glu Gln Tyr Gly
    130                 135                 140

Asp Glu Lys Asn Pro Leu Tyr Lys Tyr Tyr Glu Glu Thr Gly Asn Tyr
145                 150                 155                 160

Leu Thr Lys Tyr Ser Lys Lys Asp Asn Gly Pro Val Ile Lys Lys Ile
                165                 170                 175

Lys Tyr Tyr Gly Asn Lys Leu Asn Ala His Leu Asp Ile Thr Asp Asp
            180                 185                 190

Tyr Pro Asn Ser Arg Asn Lys Val Val Lys Leu Ser Leu Lys Pro Tyr
        195                 200                 205

Arg Phe Asp Val Tyr Leu Asp Asn Gly Val Tyr Lys Phe Val Thr Val
    210                 215                 220

Lys Asn Leu Asp Val Ile Lys Lys Glu Asn Tyr Tyr Glu Val Asn Ser
225                 230                 235                 240

Lys Cys Tyr Glu Glu Ala Lys Lys Leu Lys Lys Ile Ser Asn Gln Ala
                245                 250                 255

Glu Phe Ile Ala Ser Phe Tyr Lys Asn Asp Leu Ile Lys Ile Asn Gly
            260                 265                 270

Glu Leu Tyr Arg Val Ile Gly Val Asn Asn Asp Leu Leu Asn Arg Ile
        275                 280                 285

Glu Val Asn Met Ile Asp Ile Thr Tyr Arg Glu Tyr Leu Glu Asn Met
    290                 295                 300

Asn Asp Lys Arg Pro Pro His Ile Ile Lys Thr Ile Ala Ser Lys Thr
305                 310                 315                 320

Gln Ser Ile Lys Lys Tyr Ser Thr Asp Ile Leu Gly Asn Leu Tyr Glu
                325                 330                 335

Val Lys Ser Lys Lys His Pro Gln Ile Ile Lys Lys Gly Gly Ser Pro
            340                 345                 350

Lys Lys Lys Arg Lys Val Ser Ser Asp Tyr Lys Asp His Asp Gly Asp
        355                 360                 365

Tyr Lys Asp His Asp Ile Asp Tyr Lys Asp Asp Asp Asp Lys Ser Gly
    370                 375                 380

Gly Ser Thr Asn Leu Ser Asp Ile Ile Glu Lys Glu Thr Gly Lys Gln
385                 390                 395                 400

Leu Val Ile Gln Glu Ser Ile Leu Met Leu Pro Glu Glu Val Glu Glu
                405                 410                 415

Val Ile Gly Asn Lys Pro Glu Ser Asp Ile Leu Val His Thr Ala Tyr
            420                 425                 430

Asp Glu Ser Thr Asp Glu Asn Val Met Leu Leu Thr Ser Asp Ala Pro
        435                 440                 445

Glu Tyr Lys Pro Trp Ala Leu Val Ile Gln Asp Ser Asn Gly Glu Asn
    450                 455                 460

Lys Ile Lys Met Leu Ser Gly Gly Ser Pro Lys Lys Lys Arg Lys Val
465                 470                 475                 480

<210>  4
<211>  939
<212>  PRT
<213>  Streptococcus pyogenes

<400>  4

Met Ile Lys Ile Ala Thr Arg Lys Tyr Leu Gly Lys Gln Asn Val Tyr
1               5                   10                  15

Asp Ile Gly Val Glu Arg Asp His Asn Phe Ala Leu Lys Asn Gly Phe
            20                  25                  30

Ile Ala Ser Cys Phe Asp Ser Val Glu Ile Ser Gly Val Glu Asp Arg
        35                  40                  45

Phe Asn Ala Ser Leu Gly Thr Tyr His Asp Leu Leu Lys Ile Ile Lys
    50                  55                  60

Asp Lys Asp Phe Leu Asp Asn Glu Glu Asn Glu Asp Ile Leu Glu Asp
65                  70                  75                  80

Ile Val Leu Thr Leu Thr Leu Phe Glu Asp Arg Glu Met Ile Glu Glu
                85                  90                  95

Arg Leu Lys Thr Tyr Ala His Leu Phe Asp Asp Lys Val Met Lys Gln
            100                 105                 110

Leu Lys Arg Arg Arg Tyr Thr Gly Trp Gly Arg Leu Ser Arg Lys Leu
        115                 120                 125

Ile Asn Gly Ile Arg Asp Lys Gln Ser Gly Lys Thr Ile Leu Asp Phe
    130                 135                 140

Leu Lys Ser Asp Gly Phe Ala Asn Arg Asn Phe Met Gln Leu Ile His
145                 150                 155                 160

Asp Asp Ser Leu Thr Phe Lys Glu Asp Ile Gln Lys Ala Gln Val Ser
                165                 170                 175

Gly Gln Gly Asp Ser Leu His Glu His Ile Ala Asn Leu Ala Gly Ser
            180                 185                 190

Pro Ala Ile Lys Lys Gly Ile Leu Gln Thr Val Lys Val Val Asp Glu
        195                 200                 205

Leu Val Lys Val Met Gly Arg His Lys Pro Glu Asn Ile Val Ile Glu
    210                 215                 220

Met Ala Arg Glu Asn Gln Thr Thr Gln Lys Gly Gln Lys Asn Ser Arg
225                 230                 235                 240

Glu Arg Met Lys Arg Ile Glu Glu Gly Ile Lys Glu Leu Gly Ser Gln
                245                 250                 255

Ile Leu Lys Glu His Pro Val Glu Asn Thr Gln Leu Gln Asn Glu Lys
            260                 265                 270

Leu Tyr Leu Tyr Tyr Leu Gln Asn Gly Arg Asp Met Tyr Val Asp Gln
        275                 280                 285

Glu Leu Asp Ile Asn Arg Leu Ser Asp Tyr Asp Val Asp His Ile Val
    290                 295                 300

Pro Gln Ser Phe Leu Lys Asp Asp Ser Ile Asp Asn Lys Val Leu Thr
305                 310                 315                 320

Arg Ser Asp Lys Asn Arg Gly Lys Ser Asp Asn Val Pro Ser Glu Glu
                325                 330                 335

Val Val Lys Lys Met Lys Asn Tyr Trp Arg Gln Leu Leu Asn Ala Lys
            340                 345                 350

Leu Ile Thr Gln Arg Lys Phe Asp Asn Leu Thr Lys Ala Glu Arg Gly
        355                 360                 365

Gly Leu Ser Glu Leu Asp Lys Ala Gly Phe Ile Lys Arg Gln Leu Val
    370                 375                 380

Glu Thr Arg Gln Ile Thr Lys His Val Ala Gln Ile Leu Asp Ser Arg
385                 390                 395                 400

Met Asn Thr Lys Tyr Asp Glu Asn Asp Lys Leu Ile Arg Glu Val Lys
                405                 410                 415

Val Ile Thr Leu Lys Ser Lys Leu Val Ser Asp Phe Arg Lys Asp Phe
            420                 425                 430

Gln Phe Tyr Lys Val Arg Glu Ile Asn Asn Tyr His His Ala His Asp
        435                 440                 445

Ala Tyr Leu Asn Ala Val Val Gly Thr Ala Leu Ile Lys Lys Tyr Pro
    450                 455                 460

Lys Leu Glu Ser Glu Phe Val Tyr Gly Asp Tyr Lys Val Tyr Asp Val
465                 470                 475                 480

Arg Lys Met Ile Ala Lys Ser Glu Gln Glu Ile Gly Lys Ala Thr Ala
                485                 490                 495

Lys Tyr Phe Phe Tyr Ser Asn Ile Met Asn Phe Phe Lys Thr Glu Ile
            500                 505                 510

Thr Leu Ala Asn Gly Glu Ile Arg Lys Arg Pro Leu Ile Glu Thr Asn
        515                 520                 525

Gly Glu Thr Gly Glu Ile Val Trp Asp Lys Gly Arg Asp Phe Ala Thr
    530                 535                 540

Val Arg Lys Val Leu Ser Met Pro Gln Val Asn Ile Val Lys Lys Thr
545                 550                 555                 560

Glu Val Gln Thr Gly Gly Phe Ser Lys Glu Ser Ile Leu Pro Lys Arg
                565                 570                 575

Asn Ser Asp Lys Leu Ile Ala Arg Lys Lys Asp Trp Asp Pro Lys Lys
            580                 585                 590

Tyr Gly Gly Phe Asp Ser Pro Thr Val Ala Tyr Ser Val Leu Val Val
        595                 600                 605

Ala Lys Val Glu Lys Gly Lys Ser Lys Lys Leu Lys Ser Val Lys Glu
    610                 615                 620

Leu Leu Gly Ile Thr Ile Met Glu Arg Ser Ser Phe Glu Lys Asn Pro
625                 630                 635                 640

Ile Asp Phe Leu Glu Ala Lys Gly Tyr Lys Glu Val Lys Lys Asp Leu
                645                 650                 655

Ile Ile Lys Leu Pro Lys Tyr Ser Leu Phe Glu Leu Glu Asn Gly Arg
            660                 665                 670

Lys Arg Met Leu Ala Ser Ala Gly Glu Leu Gln Lys Gly Asn Glu Leu
        675                 680                 685

Ala Leu Pro Ser Lys Tyr Val Asn Phe Leu Tyr Leu Ala Ser His Tyr
    690                 695                 700

Glu Lys Leu Lys Gly Ser Pro Glu Asp Asn Glu Gln Lys Gln Leu Phe
705                 710                 715                 720

Val Glu Gln His Lys His Tyr Leu Asp Glu Ile Ile Glu Gln Ile Ser
                725                 730                 735

Glu Phe Ser Lys Arg Val Ile Leu Ala Asp Ala Asn Leu Asp Lys Val
            740                 745                 750

Leu Ser Ala Tyr Asn Lys His Arg Asp Lys Pro Ile Arg Glu Gln Ala
        755                 760                 765

Glu Asn Ile Ile His Leu Phe Thr Leu Thr Asn Leu Gly Ala Pro Ala

770                 775                 780

Ala Phe Lys Tyr Phe Asp Thr Thr Ile Asp Arg Lys Arg Tyr Thr Ser
785                 790                 795                 800

Thr Lys Glu Val Leu Asp Ala Thr Leu Ile His Gln Ser Ile Thr Gly
                805                 810                 815

Leu Tyr Glu Thr Arg Ile Asp Leu Ser Gln Leu Gly Gly Asp Ser Gly
            820                 825                 830

Gly Ser Thr Asn Leu Ser Asp Ile Ile Glu Lys Glu Thr Gly Lys Gln
        835                 840                 845

Leu Val Ile Gln Glu Ser Ile Leu Met Leu Pro Glu Glu Val Glu Glu
    850                 855                 860

Val Ile Gly Asn Lys Pro Glu Ser Asp Ile Leu Val His Thr Ala Tyr
865                 870                 875                 880

Asp Glu Ser Thr Asp Glu Asn Val Met Leu Leu Thr Ser Asp Ala Pro
                885                 890                 895

Glu Tyr Lys Pro Trp Ala Leu Val Ile Gln Asp Ser Asn Gly Glu Asn
            900                 905                 910

Lys Ile Lys Met Leu Ser Gly Gly Ser Pro Lys Lys Lys Arg Lys Val
        915                 920                 925

Gly Ser Tyr Pro Tyr Asp Val Pro Asp Tyr Ala
    930                 935

<210>  5
<211>  25
<212>  DNA
<213>  artificial sequence

<220>
<223>  EMX1 site1-up

<400>  5
caccgaagga cggcggcacc ggcgg                                          25

<210>  6
<211>  24
<212>  DNA
<213>  artificial sequence

<220>
<223>  EMX1 site1-dn

<400>  6
aaacccgccg gtgccgccgt cctt                                              24


<210>  7
<211>  25
<212>  DNA
<213>  artificial sequence

<220>
<223>  EMX1 site2-up

<400>  7
caccgactac gtggtgggcg ccgag                                             25


<210>  8
<211>  24
<212>  DNA
<213>  artificial sequence

<220>
<223>  EMX1 site2-dn

<400>  8
aaacctcggc gcccaccacg tagt                                              24


<210>  9
<211>  25
<212>  DNA
<213>  artificial sequence

<220>
<223>  RNF2 site1-up

<400>  9
caccggtcat cttagtcatt acctg                                             25


<210>  10
<211>  24
<212>  DNA
<213>  artificial sequence

<220>
<223>  RNF2 site1-dn

<400>  10
aaaccaggta atgactaaga tgac                                              24


<210>  11
<211>  25
<212>  DNA
<213>  artificial sequence

<220>
<223>  EMX1 site3-up

<400>  11
caccgcggat gcacggtcag cgcgg                                             25

<210>  12
<211>  24
<212>  DNA
<213>  artificial sequence

<220>
<223>  EMX1 site3-dn

<400>  12
aaacccgcgc tgaccgtgca tccg                                              24


<210>  13
<211>  25
<212>  DNA
<213>  artificial sequence

<220>
<223>  FANCF site1-up

<400>  13
caccggccgt ctccaaggtg aaagc                                             25


<210>  14
<211>  24
<212>  DNA
<213>  artificial sequence

<220>
<223>  FANCF site1-dn

<400>  14
aaacgctttc accttggaga cggc                                              24


<210>  15
<211>  35
<212>  DNA
<213>  artificial sequence

<220>
<223>  primer

<400>  15
ggagtgagta cggtgtgctc ccgcggctgc gacca                                  35


<210>  16
<211>  39
<212>  DNA
<213>  artificial sequence

<220>
<223>  primer

<400>  16
gagttggatg ctggatgggg gtgagggtag ttgagcgcc                              39


<210>  17
<211>  39

<212>  DNA
<213>  artificial sequence

<220>
<223>  primer

<400>  17
ggagtgagta cggtgtgccc cttcgcacgc aagcccaag                              39


<210>  18
<211>  41
<212>  DNA
<213>  artificial sequence

<220>
<223>  primer

<400>  18
gagttggatg ctggatggcg ggggtgatta cctgcgtctc g                            41


<210>  19
<211>  41
<212>  DNA
<213>  artificial sequence

<220>
<223>  primer

<400>  19
ggagtgagta cggtgtgcta tttccagcaa tgtctcaggc t                           41


<210>  20
<211>  45
<212>  DNA
<213>  artificial sequence

<220>
<223>  primer

<400>  20
gagttggatg ctggatgggt tttcatgttc taaaaatgta tccca                       45


<210>  21
<211>  40
<212>  DNA
<213>  artificial sequence

<220>
<223>  primer

<400>  21
ggagtgagta cggtgtgcct tcgtgagtgg cttccctgcc                             40


<210>  22
<211>  39
<212>  DNA
<213>  artificial sequence

<220>
<223>  primer

<400>  22
gagttggatg ctggatggga agaaggagtg cgggggctg                              39


<210>  23
<211>  43
<212>  DNA
<213>  artificial sequence

<220>
<223>  primer

<400>  23
ggagtgagta cggtgtgctc gaccaatagc attgcagaga ggc                         43


<210>  24
<211>  43
<212>  DNA
<213>  artificial sequence

<220>
<223>  primer

<400>  24
gagttggatg ctggatgggc tgacgtaggt agtgcttgag acc                         43